# EUROPEAN PATENT APPLICATION

(11) **EP 2 269 974 A1**
(43) Date of publication of application: **05.01.2011**
(21) Application number: 09164477.3
(22) Date of filing: 02.07.2009
(51) Int. Cl.: C07C 51/353, C07C 6/04, C07C 67/08, C07C 67/475, C07C 57/04, C07C 69/54, C07C 59/52, C07C 15/46, C07C 51/377, C12P 7/46

(54) **Bio-derived olefin synthesis**

(71) Applicant: Stichting Dienst Landbouwkundig Onderzoek, 6701 BH Wageningen (NL)
(72) Inventor: Sanders, Johan Pieter Marinus, 9722 WL Groningen (NL); van Haveren, Jacobus, 6717 XB Ede (NL); Scott, Elinor, 3822 VK Amersfort (NL); Van ES, Daniël Stephan, 6721 RP Bennekom (NL); Le Nôtre, Jörôme, 6814 BA Arnhem (NL); Spekreijse, Jurjen, 9741 JB Groningen (NL)
(74) Representative: Hatzmann, Martin

(57) **Abstract**

Disclosed is a method for the combined synthesis of at least two vinylic monomers, at least one of which being an acrylic compound, comprising subjecting a monoconjgated alkene-1- carboxylic compound to reaction with a C₂-C₄ alkene under conditions of olefin cross-metathesis. The invention is particularly useful for extracting value from protein side streams. Upon protein hydrolysis, suitable amino acids (preferably phenylalanine or tyrosine) are subjected to reductive amination so as to form the corresponding alkene-1-carboxylic acid. Preferably after esterification and separation, this is used in cross-metathesis for the concomitant production of styrene resp. hydroxy styrene, and acrylates. The invention is applicable more widely, to the synthesis of olefins on the basis of carbohydrates, naturally occurring phenolic components, natural protein resources, or amino acids obtained from fermentations.

## Description

### Field of the Invention

The invention pertains to a method of production of useful bulk chemicals from biomass resources. In particular, the invention pertains to a method for the synthesis of olefins on the basis of carbohydrates, naturally occurring phenolic components, natural protein resources, or amino acids obtained from fermentations. The invention further pertains to a reaction scheme for the carbon-retaining production of olefins from carbohydrates, naturally occurring phenolic components, natural protein resources, or amino acids obtained from fermentations.

### Background of the Invention

Traditionally, the production of organic bulk chemicals is based on petrochemical resources. As is well-known, these resources are diminishing and not renewable. Thus, based on both economical and environmental motives, people have searched for renewable sources of organic bulk chemicals.

Typical examples include the production of biodiesel from natural fatty acids e.g. from vegetable oil or bio ethanol from fermented carbohydrates from natural sources such as wheat. In comparison with the wealth of chemicals produced in the petrochemical industry, the production of useful chemicals from biomass is generally still limited in respect of the types of chemicals produced, and the extent to which biomass-derived carbon is in fact utilized in the chemical industry.

As to the types of chemicals available, it would be desired if functional chemical compounds, such as widely used vinylic monomers, particularly acrylic and styrenic monomers, could be produced from biomass, i.e. from plant or animal derived materials.

As to the utilization of biomass-derived carbon, it would be desired if functional chemicals, notably monomers as mentioned hereinbefore, could be produced in which the majority of carbon atoms, and preferably all carbon atoms, originate from biomass carbon. Particularly, it would be desired to achieve this at a high yield.

A reference in this respect is WO 2009/045673. Herein a method is described of manufacturing acrylic acid by reacting fumaric acid with ethylene in the presence of a cross-metathesis transformation catalyst. The fumaric acid typically is obtained through biosynthesis from a source of glucose. The process is entirely focused on utilizing fumaric acid, and is limited to the production of acrylic acid and/or esters thereof.

A desire behind the present invention is to provide a more widely useful method to convert bio-derived carbon (i.e. not necessarily from glucose and not necessarily through fumaric acid) to useful functional chemicals (i.e. not necessarily limited to acrylic acid).

### Summary of the Invention

In order to better address one or more of the foregoing desires, the invention, in one aspect, is a method for the combined synthesis of at least two different vinylic monomers, at least one of which being an acrylic compound, comprising subjecting a monoconjugated alkene-1- carboxylic compound to reaction with a C₂-C₄ alkene under conditions of olefin cross-metathesis.

In another aspect, the invention presents the use of an olefin cross-metathesis reaction between a C₂-C₄ alkene and a monoconjugated alkene-1-carboxylic compound, for the purpose of producing vinyl compounds from biomass, particularly biomass comprising functional olefin compounds.

In yet another aspect, the invention is a method for the combined synthesis of at least two different vinyl compounds from a hydrolyzed protein source, comprising the steps of
(a) subjecting the hydrolyzed protein to reductive deamination so as to produce at least one carboxylic acid functional alkene;
(b) optionally, though preferably, subjecting the at least one carboxylic acid functional alkene to esterification with an alcohol so as to form at least one carboxylic ester functional alkene;
(c) subjecting the at least one carboxylic acid or ester to olefin cross metathesis in the presence of a C₂-C₄ alkene.

### Description of the Drawings

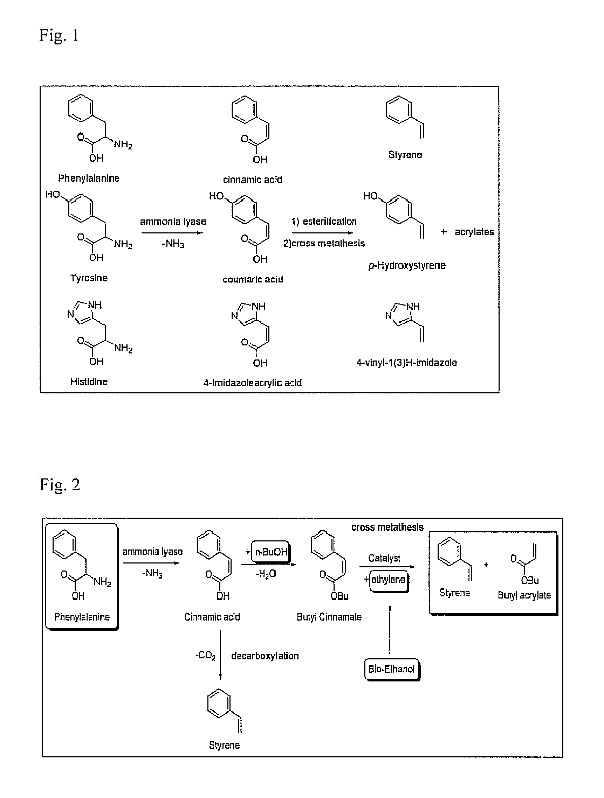

Fig. 1 depicts preferred amino acids, the acids that result from their reductive deamination, and the vinyl compounds that result from their subjection to olefin cross-metathesis.
Fig. 2 depicts an overall scheme for the production of styrene and butyl acrylate from phenylalanine. The (reference) production of styrene from cinnamic acid through decarboxylation is also depicted herein.

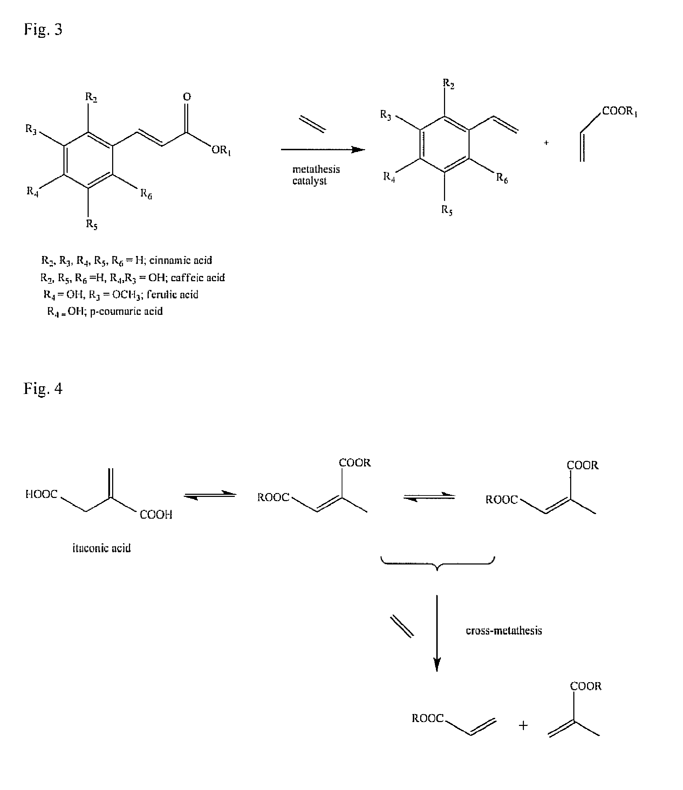

Fig. 3 depicts a more general scheme for the cross-metathesis based on not only cinnamic acid, but also caffeic acid, ferulic acid, and p-coumaric acid.
Fig. 4 depicts a scheme for the production of acrylic acid and methacrylic acid based upon carbohydrate derived itaconic acid.

### Detailed Description of the Invention

In a general sense, the invention is based on the insight to optimize the availability of functionalized hydrocarbon compounds from biomass, by producing a mixture of at least two of such functional compounds. Without wishing to be bound by theory, the inventors believe that this represents a counter-intuitive measure in chemistry.

One element in realizing the foregoing is the use of olefin cross-metathesis as a synthesis tool. It will be apparent to the skilled person that such cross-metathesis is in itself known. In fact, the olefin metathesis reaction has become a powerful weapon for the coupling of carbon-carbon double bonds. Drs. Grubbs, Schrock, and Chauvin shared the 2005 Nobel Prize in Chemistry for the development of the olefin metathesis reaction. The generally accepted mechanism for the olefin metathesis reaction involves a metal carbene acting as a catalyst to metathesize two alkenes into a new alkene through a metallocyclobutane intermediate. The newly synthesized alkene contains one methylene carbon from each of the two starting alkenes. Olefin metathesis catalysts developed by Schrock, Grubbs, and others are commercially available, making the olefin metathesis reaction a viable and useful strategy in organic chemistry. Examples of commercially available olefin metathesis catalysts include the "Schrock catalyst" (i.e., [Mo(=CHMe_{2Ph})(=N-Ar)(OCMe(CF₃)₂)₂], the "1st generation Grubb's catalyst" (i.e., [Ru(=CHPh)Cl₂(PC_{y3})₂], and the "2nd generation Grubb's catalyst" (i.e., [Ru(=CHPh)Cl₂PCy₃(N,N'-diaryl-2-imidazolidinyl)] (Me=methyl, Ph=phenyl, Ar=aryl, and Cy=cyclohexyl).

Olefin cross-metathesis is used with (poly)unsaturated fatty acid acids or esters, with ethene, in making alpha olefins of reduced chain length. See e.g., WO 2004/062763. The reaction is also used as a polymerization mechanism.

In the present invention, the olefin cross-metathesis reaction is used as a synthetic step in extracting value (in terms of functional, useful vinylic compounds) from chemicals available in biomass. It should be noted that regular thinking of obtaining useful chemicals from biomass mainly involves separation and isolation of available functional chemicals. In the present invention another chemical, viz. a C₂-C₄ alkene and, preferably, ethene, is added so as to enable an even better yield of useful chemicals from biomass.

In a preferred embodiment, so as to provide a process that overall used biomass-derived carbon, the ethene is "bio-ethene" e.g. produced from bioethanol. In the event that, e.g., propylene or butylene is used, these alkenes too are preferably biobased.

In the above-described method, the olefin cross-metathesis is put to use with a monoconjugated alkene-1- carboxylic compound. The term "monoconjugated alkene-1-carboxylic compound" is used to indicate that, irrespective of any further carboxylic groups and/or double bonds in the same molecule, and irrespective of any multiple conjugations (like between several carbon-t-carbon double bonds, or between double bonds and aromatic rings) the carboxylic compound has only one double bond conjugated with a carboxylic C=O double bond. The term "conjugated" is known to the skilled person in organic chemistry. In the context of a monoconjugated alkene-1-carboxylic acid it refers to the presence of a carbon-to-carbon double bond (i.e. a C=C bond) and a carboxylic carbon-to-oxygen double bond (i.e. the C=O bond in a carboxylic functional group) that are covalently linked to each other via a single carbon-carbon bond. Or, in other words, one carbon atoms of a single covalent carbon-carbon bond is covalently attached to a further carbon atom through a double bond, and the other carbon atom of the same single covalent carbon-carbon bond is covalently attached to an oxygen through a double bond. This conjugated system can be depicted as -C=C-C=O-. Preferably, the monoconjugated alkene-1-carboxylic compounds are selected from the group consisting of alkene-1- mono carboxylic compounds, asymmetric alkene-1-dicarboxylic compounds, and mixtures thereof.

The foregoing compounds also known as "enoic acids." These enoic acids (and carboxylic derivatives thereof such as esters or amides) can be made well available from biomass. A particular source is in proteins, such as obtainable as a side stream (e.g. dried distiller grains with solubles (DDGS) from wheat or the non-dried form of DDGS or other distilled grains, rape and soya cake, proteins derived from grasses or algae or seaweeds).

Thus, in another aspect of the invention, such side stream proteins are subjected to hydrolysis so as to yield amino acids. Protein hydrolysis, e.g. enzymatic hydrolysis is well-known and generally available to the skilled person.

The hydrolysis is to be conducted so as to obtain one or more amino acids that, upon the reductive deamination of the amino group vicinal to the carboxylic acid group, will result in the formation of an alkene-1-carboxylic acid. The reductive deamination preferably involves using an ammonia lyase. Whilst most amino acids will, in principle, be able to result in an alkene-1-carboxylic acid upon deamination with release of ammonia, it will be understood that this does not hold for glycine (2-amino acetic acid), as this lacks the further carbon atom through which, upon deamination, a double bond could be formed.

Preferred amino acid, in terms of more optimally facilitating the formation of the aforementioned C=C double bond, are those in which the new double bond will be conjugated with an existing unsaturated, preferably aromatic, structure. These preferred amino acids are: tyrosine, phenyl alanine, methyl aspartate, serine, and histidine. Other preferred amino acids include aspartic acid, and (deaminated) asparagine, which are capable of yielding two acrylic acid molecules per molecule of amino acid. Still other preferred amino acids include glutamic acid and (deaminated) glutamine. The latter amino acids are capable of enzymatic conversion (using mutanase) into methyl aspartate which, in turn, is capable of yielding methyl acrylate and acrylate under the influence of ammonium lyase.

With a view to the production of useful bulk chemicals, particularly monomers, those amino acids are preferred that, upon olefin cross-metathesis, will form a useful vinyl compound. Thus, further preferred amino acids are phenylalanine and tyrosine, which yield styrene resp. 4-hydroxystyrene.

It should be noted that the use of phenylalanine as a source of styrene (and equally the use of tyrosine as a source of p-hydroxy styrene, is known. Known processes, e.g. as described in WO 2005/116229, typically involve decarboxylation, with release of CO₂, and thus do not optimally use the mass available from existing biomolecules.

The invention, on the other hand, by employing olefin cross metathesis, allows the carboxyl moiety to end up in a useful molecule as well.

To this end the alkene-1-carboxylic compound is subjected to olefin cross-metathesis To this end the carboxylic compound is first isolated from its biomass origins. Particularly, upon the preferred use of amino acids from protein hydrolyzates, the desired deaminated amino acid is isolated. It should be noted that it is possible to first isolate, from the protein hydrolyzate, any desired amino acid and then subject the amino acid to reductive deamination. However, considering the chromatographic separation techniques available for individual amino acids from protein hydrolyzates, and the available enzymatic specificity for conducting reductive deamination, it is preferred to just subject the protein hydrolyzate to the deamination.

Thus, after hydrolysis of a protein side stream, the desired amino acids, and preferably the most interesting ones (e.g. Tyr, Phe, His, Glu and Asp) will be converted by elimination of ammonia to the corresponding 2-enoic acids ("reductive deamination") through the action of carbon-nitrogen lyases or any other suitable reagent. For instance, phenylalanine ammonia-lyase (EC 4.3.1.5) catalyzes the conversion of L-phenylalanine (and L-tyrosine) into *trans*-cinnamate (and trans-coumarate), whereas histidine ammonia lyase (EC 4.3.1.3) converts histidine into urocanate. Ammonia lyases are available commercially.

As a result of deamination, amino acids will loose their zwitterionic character resulting in different chemical and physical properties, which facilitates separation.

To further enhance separation, it is preferred for the alkene-1-carboxylic acids formed to be esterified. In some cases the acids will be sufficiently apolar to be extracted into an immiscible organic media, in which they can be easily esterified with alcohols such as ethanol or butanol. Esterification may be carried out with the aid of esterases or by conventional chemical means such as the use of (novel) acidic catalysts. In the case of phenylalanine the solubility in aqueous media is higher than that of the resultant deaminated product cinnamic acid. Here it will be preferred to conduct solid-liquid separation of the cinnamic acid prior to esterification.

Whilst esterification thus optimizes the possibilities to obtain an isolated alkene-1-carboxylic compound as a starting material for subsequent olefin cross-metathesis, esters usually result in higher yield in cross-metathesis reactions than free carboxylic acids and the esters are further preferred in view of the end-products obtainable therewith. Many acrylate monomers are used in the form of esters, and it would be desirable to directly produce such esters. Thus, preferably, the alkene-1-carboxylic compound is an alkene-1-carboxylic ester, preferably an ester of a straight-chain or branched alcohol having up to 10 carbon atoms, more preferably having 1 to 4 carbon atoms, and most preferably methanol, ethanol or n-butanol. It will be understood that, if the 1-alkene carboxylic compound is a free carboxylic acid, also the resulting acrylic compound will be an acrylic acid.

With reference to the aforementioned preferred amino acids and the resulting deamination products thereof, the most preferred esters are methyl, ethyl, and n-butyl cinnamate, as well as methyl, ethyl, and n-butyl coumarate.

In olefin cross-metathesis with a C₂-C₄ alkene, these esters will result in the co-production of an acrylate (carrying the same ester group) and an olefin carrying, directly attached to the double bond therein, the residue of the amino acid that has resulted in the acrylate. It will be understood that in the event of other alkene-1-carboxylic compounds, such as the free acid or any other carboxylic modifications available in the art (e.g. amides), the result will, *mutatis mutandis,* be the same as with the esters.

The alkene added in the cross-metathesis, preferably is ethene. If ethene is used, the resulting compound will be an alpha-olefin, i.e. a vinyl compound having a terminal alkenyl group. If, e.g., 1-propene or 1-butene is used, the resulting vinyl compound, on one end of the double bond, will carry the aforementioned amino acid residue, and on the other end will carry the residue of the added alkene (i.e. methyl in the case of 1-propene or ethyl in the case of 1-butene). In the event of using ethene, the result is a vinyl compound having a terminal alkene group (i.e. an alpha-olefin), attached to the residue of the original amino acid. E.g. in the preferred example of phenylalanine, this result in this case will be styrene.

With reference to the preferred use of phenyl alanine or tyrosine, a preferred embodiment of the method according to the invention is as follows. Herein the alkene-1- carboxylic compound is selected from the group consisting of the methyl ester of cinnamic acid, the ethyl ester of cinnamic acid, the butyl ester of cinnamic acid, the methyl ester of coumaric acid, the ethyl ester of coumaric acid, the butyl ester of coumaric acid, and mixtures thereof,. Taking into account the further preference that the C₂-C₄ alkene is ethene, as a result one of the two vinyl compounds produced is styrene or 4-hydroxy styrene, and the other is methyl, ethyl, or butyl acrylate.

The olefin cross-metathesis can be carried out with reference to known catalysts and conditions. Typical catalysts are metal carbene complexes of tungsten, molybdenum, rhenium, chromium, osmium and ruthenium. Examples of metathesis catalyst composition, processes and products are described in the Handbook of Metathesis, R.H. Grubbs Ed. Wiley VCH- ISBN 3-527-30616-1.

The invention also pertains to the use of an olefin cross-metathesis reaction between a C₂-C₄ alkene, preferably ethene, and a monoconjugated alkene-1- carboxylic compound, for the purpose of producing vinyl compounds from biomass comprising such alkene-1- carboxylic compounds, or precursors for these compounds. With reference to the above, a good example of these precursors are proteins. Other biomass precursors or direct sources of alkene-1-carboxylic compounds, useful in producing two different vinyl monomers, include fermentation products of carbohydrates such as itaconic acid, citraconic acid or mesaconic acid. These are all asymmetric dicarboxylic acids and therewith capable of yielding two different vinyl monomers upon olefin cross-metathesis. E.g. fumaric acid, as used in WO 2009/045637 is not a monoconjugated alkene-1-carboxylic compound, but a symmetric biconjugated alkene-1-carbocylic compound (having a carbon to carbon double bond conjugated with two carboxylic carbon to oxygen double bonds). Other possible monoconjugated alkene-1-carboxylic acids are naturally occurring phenolic components such as ferulic acid, cinnamic acid and caffeic acid. But also other alpha hydroxyacids can be good substrates. E.g. hydroxybutyric acid after dehydration, or upon depolymerization of polyhydroxybutyrate. This also holds for other hydroxy acids of bacterial origin, e.g. for alkene-1-carbocylic acids that can be obtained via hydrolysis of poly(hydroxy alkanoates), such as poly(3-hydroxyvalerate), poly(4-hydroxyvalerate), poly(4-hydroxy buyrate), poly(4-hydroxycaproate), and the like, and dehydration so as to introduce a conjugated double bond (by which an alkene-1-carboxylic acid is formed).

According to the invention, the judicious choice of olefin cross metathesis as a synthesis tool serves to extract more value from the biomass, by utilizing a higher percentage of biomass-derived carbon (e.g. as opposed to decarboxylation). This is particularly realized by the insight to produce two useful chemicals together. It will be understood that the starting materials almost by definition comprise a plurality of different compounds. Whilst the present invention will not generally lead to using up all of these different compounds, it provides essential tools to obtain two end-products at a time, instead of one.

The cross-metathesis process leads to a highly attractive and unique coupled production of two bulk chemicals e.g. styrene + butylacrylate or butylacrylate +methacrylic acid. As the components of these mixtures (and the excess ethylene) have all distinct boiling points, separation can be accomplished by established distillation techniques. In an alternative embodiment, the combined produced monomers can be used, directly, or after partial purification, to produce copolymers.

A preferred biomass resource for alkene-1-carboxylic compounds is formed by proteins, and particularly side-stream proteins, as referred to above. For these resources, the present invention not only adds value by the production of two useful vinyl monomers, it does so in a way not depriving the rest of the biomass from other useful applications. E.g. if only phenyl alanine, using a preferred embodiment of the present invention, is detracted from the protein hydrolyzate, the residue of the hydrolyzate will still comprise all of the other amino acids, and will thus still be valuable e.g. as animal feed. Further, also the ammonia resulting from the reductive deamination of amino acids as described above, is a widely used bulk chemical.

As to the use of protein biomass, another element of the invention provides a particular advantage, and that is the esterification as mentioned above. Thus, all in all, in a preferred embodiment, the invention includes a method for the combined synthesis of at least two different vinyl compounds from a hydrolyzed protein source, comprising the steps of
(a) subjecting the hydrolyzed protein to reductive deamination so as to produce at least one carboxylic acid functional 1-alkene;
(b) subjecting the at least one carboxylic acid functional 1-alkene to esterification with an alcohol so as to form at least one carboxylic ester functional 1-alkene;
(c) subjecting, preferably after separation of the ester, the at least one carboxylic acid ester to olefin cross metathesis in the presence of a C₂-C₄ alkene.

With reference to the above discussed preferred embodiments, the carboxylic acid functional 1-alkene preferably is cinnamic acid (as based on phenylalanine obtained from protein hydrolysis) or coumaric acid (as based on tyrosine obtained from protein hydrolysis).

With reference to using side stream proteins from biofuel production, and ethene obtained via bioethanol, the present invention preferably provides an overall, integrated, chemical manufacturing based on starting materials from biological resources.

Thus, in a preferred embodiment, the invention allows to convert sidestream proteins derived from biofuels and other industrial processes, but also amino acids and alpha hydroxyacids produced by fermentation or directly in plants, into acrylic and styrenic bulk chemicals with co-production of feed and ammonia. The side stream of biofuel production preferably is from wheat, corn, cassava, soy, rape seed, sunflower, palm, beet, cane, grass, Luzerne, algae, cyanobacteria, miscanthus, switchgrass, slaughterhouse waste, or manure.

Applying a cross metathesis reaction on deaminated amino acids with ethylene (derived from bioethanol) in order to produce styrenics and acrylates provides a high atom economy (compared to e.g. decarboxylation of cinnamic acid to styrene). High value products like acrylates thus can be generated from large volume low cost bio-based chemicals (phenylalanine, bio-ethanol, bio-butanol).

Besides being an amino acid obtainable by the hydrolysis of a natural protein source, the 1-carboxylic acid can also be an amino acid obtained by fermentation of carbohydrates, or another acid obtained from fermentation, such as a hydroxy acid.

Although it is preferred to obtain the acids from natural resources, it is also possible to use amino acids obtained by chemical synthesis. This has the advantage of not being limited to natural amino acids, i.e. to use the synthetic freedom available to the skilled person to design and produce amino acids attuned to the production of suitable olefins by cross metathesis. Another option is to harvest suitable amino acids from plants that are genetically modified so as to produce the amino acid.

## Claims

1. A method for the combined synthesis of at least two different vinylic monomers, at least one of which being an acrylic compound, comprising subjecting a monoconjugated alkene-1- carboxylic compound to reaction with a C₂-C₄ alkene under conditions of olefin cross-metathesis.

2. A method according to claim 1, wherein the C₂-C₄ alkene is ethene.

3. A method according to claim 1 or 2, wherein the monoconjugated alkene-1- carboxylic compound is an ester, preferably an ethyl or n-butyl ester.

4. A method according to any one of the preceding claims, wherein the monoconjugated alkene-1- carboxylic compound is obtained by the reductive deamination of an amino acid.

5. A method according to claim 4, wherein the amino acid is obtained by the hydrolysis of a natural protein source.

6. A method according to claim 5, wherein the protein source is a side stream of biofuel production, preferably from wheat, corn, cassava, soy, rape seed, sunflower, palm, beet, cane,grass, Luzerne, algae, cyanobacteria, miscanthus, switchgrass, slaughterhouse waste, or manure.

7. A method according to claim 4, wherein the amino acid is obtained by fermentation of carbohydrates.

8. A method according to claim 4, wherein the amino acid is obtained by chemical synthesis or by harvesting from plants that are genetically modified so as to produce the amino acid.

9. A method according to any one of the claims 1-3, wherein the monoconjugated-1-carboxylic acid is selected from the group consisting of itaconic acid, citraconic acid, mesaconic acid, ferulic acid, cinnamic acid, and caffeic acid, preferably obtained from the fermentation of carbohydrates.

10. A method according to any one of the preceding claims, wherein the C₂-C₄ alkene is ethene obtained from bioethanol.

11. A method according to any one of the preceding claims, wherein the alkene-1- carboxylic compound is selected from the group consisting of the methyl ester of cinnamic acid, the ethyl ester of cinnamic acid, the butyl ester of cinnamic acid, the methyl ester of coumaric acid, the ethyl ester of coumaric acid, the butyl ester of coumaric acid, and mixtures thereof, wherein the C₂-C₄ alkene is ethene, and wherein one of the two vinyl compounds produced is styrene or 4-hydroxy styrene, and the other is methyl, ethyl, or butyl acrylate.

12. The use of an olefin cross-metathesis reaction between a C₂-C₄ alkene, preferably ethene, and a monoconjugated alkene-1- carboxylic acid, for the purpose of producing vinyl compounds from biomass comprising such alkene-1- carboxylic compounds, or precursors for these compounds.

13. A use according to claim 11, wherein the biomass is a protein side-stream.

14. A method for the combined synthesis of a vinyl compound from a hydrolyzed protein source, comprising the steps of
(a) subjecting the hydrolyzed protein to reductive deamination so as to produce at least one carboxylic acid functional 1-alkene;
(b) subjecting the at least one carboxylic acid functional 1-alkene to esterification with an alcohol so as to form at least one carboxylic ester functional alkene;
(c) subjecting, preferably after separation of the ester, the at least one carboxylic acid ester to olefin cross metathesis in the presence of a C₂-C₄ alkene, preferably ethene.

15. A method according to claim 13, wherein the carboxylic acid functional 1-alkene is selected from the group consisting of cinnamic acid, coumaric acid, caffeic acid, ferulic acid and mixtures thereof.
